Europäisches Patentamt

European Patent Office

Office européen des brevets

(19)

(11) **EP 1 586 546 A2**

(12) **EUROPEAN PATENT APPLICATION**

(43) Date of publication:
**19.10.2005  Bulletin 2005/42**

(51) Int Cl.⁷: **C04B 14/06**, G01N 11/00,
G01N 33/00

(21) Application number: **05075858.0**

(22) Date of filing: **14.04.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **14.04.2004  NL 1025948**

(71) Applicant: **Ballast Maatschappij "De Merwede" B.V.**
**6019 AA Wessem (NL)**

(72) Inventor: **Van Mechelen, Dirk Maria Alfons Jules**
**2830 Tisselt (BE)**

(74) Representative:
**Jennen, Peter Leonardus Hendricus**
**Exter Polak & Charlouis B.V.,**
**P.O. Box 3241**
**2280 GE Rijswijk (NL)**

(54) **Method and device for composing a sand mixture for concrete, and sand mixture and concrete thus obtained**

(57)     The invention relates to a method and device for composing fine aggregates for concrete, to fine aggregates composed in this way and concrete produced therewith, and also to methods for determining the conductivity ratio of a sand and for measuring viscosity of concrete produced therewith.

The invention is based on the insight that the conductivity ratio of a sand, i.e. the electrical conductivity of a mixture of the said sand in a liquid divided by the electrical conductivity of the liquid, is a significant indicator for predicting the viscosity properties of concrete produced using the corresponding sand. Matching the conductivity ratio to an ideal value for the said sand also allows the viscosity to be minimized. The conductivity ratio can be matched, for example, by mixing with another suitable sand grade, in particular a fine sand grade, or altering the salt content.

EP 1 586 546 A2

**Description**

A first aspect of the present invention relates to a method for composing a mixture of fine aggregates for concrete.

Furthermore, the invention relates to a sand mixture obtainable in this way, and to a method for producing concrete (mortar) using the sand mixture, and to concrete (mortar) obtainable in this way.

Moreover, the invention relates to a method for measuring a conductivity ratio of a sand, and to a method for determining concrete viscosity as a function of a conductivity ratio of a sand grade.

In addition, the invention relates to a device for composing fine aggregates for a concrete.

Concrete is a construction material in very widespread use. Concrete generally comprises cement, aggregates, water and often, although not necessarily, auxiliaries and additions. Aggregates are in turn subdivided into coarse and fine aggregates. The coarse aggregates comprise the "gravel", and the find aggregates comprise the "sand". In the context of the present invention, it is always only fine aggregates which are under discussion. It will be implicitly understood that it is in principle also possible for coarse aggregates to be added to the concrete; in this context, concrete without coarse aggregates is also referred to as concrete mortar.

The "sand" can be classified into different degrees of fineness, such as 0/4 mm, 0/2 mm and 0/1 mm, on the basis of the grain size and grain size distribution, for example in accordance with NEN 5905.

Concrete is required to satisfy numerous regulations relating to the quality, strength, etc. Inter alia the VBT [Dutch Concrete Technology Regulations] impose demands on the grain distribution of the coarse and fine aggregates in concrete. In addition, industry imposes demands on the concrete relating, inter alia, to its workability.

In practice, this has led to a rule that the 0/4 mm sand forms the main constituent of the fine aggregates. (Furthermore, a content of from 115 to 140 litres/m$^3$ of material which is finer than 250 $\mu$m (cement plus sand), depending on the maximum grain size in the mixture, is prescribed for concrete.) Furthermore, the maximum fraction smaller than 1 mm may be exceeded, provided that additional requirements relating to the water cement ratio and the minimum cement content are satisfied. In general, a sand mixture for concrete does not in principle have to satisfy the standard if it is demonstrated that the concrete made using the corresponding sand mixture does comply with the standard(s) for concrete.

One problem in practice has proven to be that the workability of a concrete is difficult to predict as a function of the composite parts, in particular as a function of the sand grade. Workability, a term which is used as a measure of the viscosity of concrete, is also an indication of the strength which can possibly be achieved, as well as other properties of the concrete. By way of example, concrete with a good workability, i.e. a low viscosity, expressed, for example, in Ncm, can be vibrated well. This indicates that the strength which can ultimately be achieved with the concrete can be very good. If the workability of a concrete could be improved, this can have a beneficial effect on the strength of the set concrete, or less water and less cement could be used to achieve the same ultimate strength, etc.

More generally, the workability (viscosity) is an important parameter for concrete. When producing a concrete, at least three types of materials are mixed, namely cement, water and at least fine aggregates, as has already been indicated above. In practice, it is often possible to select from numerous types of aggregates. The influence of these materials on the workability has not hitherto been adequately predictable.

In practice, therefore, there is a demand for a method for composing fine aggregates for concrete in which it is possible to make a prediction about the workability of the concrete obtained using these aggregates.

To this end, the invention provides a method according to claim 1. In particular, a first aspect of the invention provides a method for composing a mixture M of fine aggregates for concrete, in which the fine aggregates comprise one or more sand grades with a grain size of substantially 0/4 mm or below and zero or more optionally dissolved salts, excluding cement, the method comprising the steps of providing a first sand grade Z1, determining a conductivity ratio FF(Z1) of Z1, the conductivity ratio being the mathematical ratio between a specific electrical conductivity of a substantially insulating liquid and a specific electrical conductivity of the sand grade in this liquid, selecting a range of values for usable conductivity ratios FFb(Z1) associated with the sand grade Z1, matching a composition of the fine aggregates to a mixture M with a conductivity ratio FF(M), by adding or removing at least one additional fine aggregate to or from the sand grade Z1, in a ratio which is such that the FF(M) of the mixture M is within the selected range of values of usable conductivity ratios FFb (Z1) .

In this context, a conductivity ratio FF(Z1) of Z1 can be determined, for example, with the aid of a method and/or device according to another aspect of the present invention, by looking in previously compiled tables and so on. It is important to note that it is therefore not necessary to determine electrical conductivities on site, but rather it is sufficient for the electrical conductivity to be known at the time of composing the mixture.

There are no particular restrictions on the selection of a range of values of usable conductivity ratios FFb(Z1) associated with the sand grade Z1, and the choice may be governed, for example, by a desired (usable) workability of the concrete to be produced. The conductivity ratio associated with this workability may, for example, be or have been determined by tests. Alternatively, tables produced previously can also be used for this purpose. It is also possible, for example, to keep the conductivity ratio the same, so that FFb(Z1) coincides with FF(Z1). Other selection criteria are possible.

The invention is based on the inventor's discovery that the conductivity ratio of sand is a variable which can be used to predict the workability of concrete produced therewith, and in particular to predict the effects on the workability of concrete if the composition is altered. Moreover, it has been found that other properties, such as durability, are not adversely affected or are even positively influenced.

There now follows below some more extensive background information. Hitherto, it has been assumed that the addition of, for example, fine sand to concrete was always detrimental to the workability, since it was believed that the larger grain surface area of the fine sand grains increased the demand for water in the concrete mixture. Extensive tests, as described, for example, in Report 2003-2, "fine sand in concrete", by the CUR Institute, confirmed this belief, and it always appeared necessary either to add more water or to add a so-called super-plasticizer to the concrete mixture. Another point is that hitherto it did not readily appear possible to predict what happens if two sand grades are mixed, or why sea sand often cannot be used, and indeed if it can be used why washing it (in order to lower the salt content) can have such an adverse effect.

As has been stated, the inventor has discovered that the conductivity ratio of the aggregates used is a variable which can be used to predict these properties. One way of determining this ratio is explained in more detail below, but for the moment it suffices to say that the resistivity of a mass of sand in a liquid such as water is divided by the resistivity of this liquid. One condition for a measurement to be usable is for the liquid to be a good insulator. The conduction which is still measured is then the conduction between grains which are in contact with one another.

It should be noted that the conductivity ratio referred to here is also known as the "formation factor", or FF for short. This conductivity ratio can in practice also be replaced by its reciprocal, also known as the "compatibility factor" or CF for short, by a few simple arithmetic adjustments to formulae and tables. The conductivity ratio measured in practice has been measured for a number of sand grades, and its reciprocal $1/FF(Z1)$, i.e. the CF(Z1) thereof, is given in Table 1, column 2.

According to the invention, if the conductivity ratio of the fine aggregates is altered by whatever measure, the workability of concrete produced therewith will also change, even if the other constituent parts and the mixing ratio (in % by mass) of fine aggregates in the concrete do not change. Obviously, it is possible that the workability will by chance turn out to be the same value again, but in that case the viscosity will pass through an at least local extreme.

According to the invention, it will also be the case that, if the conductivity ratio remains within a defined range of values, the workability of the concrete containing the fine aggregates remains at least as good, i.e. the viscosity does not increase. In this context, other changes to the concrete, for example a different water cement ratio, have not proven to have any significant influence on the change. In other words, if a mixture of fine aggregates is worse than the pure sand grade Z1, this relative difference is in principle present in any other concrete produced using the said mixture of fine aggregates compared to concrete produced using the pure sand grade Z1.

It is in principle unimportant how this range of values of conductivity ratios has been determined. It can be determined by tests, for example in a manner according to the invention which will be explained below. The range can also be determined on the basis of tests on the workability of the concrete which has been produced using various mixtures. However, the method according to the invention makes it possible to predict what mixtures may and indeed will be suitable. This is therefore a major advantage of the invention, namely that it is possible to give formulations for good mixtures of fine aggregates, or even for treatments of sand grades required to give better concrete properties.

The method according to the invention can be used, for example, to search for a sand composition for concrete with a better workability or for a sand composition for concrete with properties which are as good, on the basis of sand grades of which there is ample availability but which are seldom or never used. All this will be explained in more detail below.

The following comments are made in connection with the term "fine aggregates". In principle, the fine aggregates comprise the sand, i.e. the one or more sand grades present, with a grain size distribution in the category 0/5 mm in accordance with NEN 5905 or finer. By way of example, this does not include gravel. In the context of the present application, this term also does not encompass cement or fly ash or other fillers. However, it can include salts, provided that they are not super-plasticizers or retardants. It should be noted that sand may itself already comprise salt, for example in the case of sea sand, which contains NaCl. In this case, therefore, by way of example it is possible for NaCl to be removed from the sea sand. More generally, it is therefore possible to add one or more substances to a fine aggregate, but also for one or more substances to be removed from it, this latter option applying specifically to (soluble) salts.

In particular, FFb(Z1) is the range of values of usable conductivity ratios in which the viscosity of a concrete produced using fine aggregates which comprise at least 50% by mass of Z1 is at least equal to the viscosity of an otherwise identical concrete produced using fine aggregates consisting exclusively of Z1. This means that concrete with a large proportion of Z1 in the fine aggregates has a viscosity which is dependent on the conductivity ratio of the fine aggregates. The conductivity ratio of the fine aggregates can be altered with respect to the conductivity ratio of pure Z1, in which case the influence on the viscosity can be determined by measuring the latter for the concrete produced using the altered fine aggregates. It is in this way possible to determine a range of values of conductivity ratios for the fine aggregates with which the concrete has a viscosity which is at the equal of, i.e. at least as low as, concrete comprising pure Z1. It should be noted that this does not exclude the possibility of the level of Z1 in the fine aggregates being lower than 50% by mass, but obviously the importance of a correctly selected conductivity ratio in the case of a (very) low level of Z1 will be lower than the importance of a correctly set conductivity ratio of other fine aggregates.

Sand grade Z1 is preferably selected from Table 1, column 1, and 1/FFb(Z1) is preferably selected from Table 1, column 3. The range of usable conductivity ratios has been determined for a large number of sand grades that are in widespread use. If the conductivity ratio changes as a result of another fine aggregate being added to or removed from Z1, but is still within the range of values of usable conductivity ratios, the viscosity of otherwise identical concrete produced using the modified fine aggregates will at least remain the same or will become lower. It should be noted that if desired it is possible to set the conductivity ratio to a value outside the abovementioned range of usable conductivity ratios. The viscosity will then be higher, and the workability of concrete worse, but in some cases this could be desirable.

The difference between FF(M) of the mixture and a predetermined range of values of desirable conductivity ratios FFw(Z1) associated with sand grade Z1 is advantageously at most equal to and more advantageously lower than the difference between the FF(Z1) and FFw(Z1), and more advantageously still is virtually 0.

In particular, FFw(Z1) is the range of values of conductivity ratios at which the viscosity of a concrete produced using fine aggregates comprising at least 50% by mass of Z1 is at least 10%, and preferably at least 25%, lower than the viscosity of an otherwise identical concrete produced using fine aggregates consisting exclusively of Z1. A range of values of desired conductivity ratios associated with sand grade Z1 can be determined in a similar way to that which has been described above for the range of values of usable conductivity ratios. In virtually all cases, it is possible to obtain a lower viscosity of concrete produced using the modified fine aggregates. Often, even a much lower value is possible. Examples in this respect are given below. As has already been described, a lower viscosity has advantages relating to the properties of the set concrete. A lower viscosity makes it possible to vibrate the concrete more successively, which has a positive effect on the density and ultimately the strength which can be achieved. It is also possible to add less water, which increases the viscosity but such that it is possible to obtain a concrete with, for example, the same viscosity as the concrete containing pure Z1 which, however, also has a higher density and strength.

It is advantageous for sand grade Z1 to be selected from Table 1, column 1, and for 1/FFw(Z1) to be selected from Table 1, column 4. A range of values of desirable conductivity ratios has been determined for a number of sand grades. As has been stated, these are given in Table 1, column 4. In this case, the boundaries for the range of values 1/FFw are determined on the basis of the criterion that the viscosity of the concrete mixture with a modified conductivity ratio was at least 10% lower compared to the unmodified concrete mixture. Obviously, it would also be possible to apply other criteria, for example a greater relative change, such as a 30% lower viscosity, or alternatively a defined maximum value for the viscosity. The boundaries of the range of values FFw then also change on the basis of these different criteria.

It is preferable for the difference between FF(M) of the mixture and a predetermined ideal value of the conductivity ratio FFi(Z1) associated with the sand grade Z1 to be at most equal to and advantageously lower than the difference between FF(Z1) and FFi(Z1) . An ideal value for the conductivity ratio can be found for each sand grade. Occasionally, there are two or more values with either more or less the same viscosity achievable or a clear local minimum. If the difference between the conductivity ratio as set for the fine aggregates and the conductivity ratio which is ideal for this aggregate becomes lower, the viscosity of the concrete produced therewith will generally also become lower. In exceptional cases, the viscosity passes through a local maximum as a function of the conductivity ratio between a conductivity ratio of Z1 and the ideal conductivity ratio of Z1.

It is advantageous for the difference between FF(M) and FFi(Z1) to be less than 5% of FFi(Z1). With such minor differences in conductivity ratios, the viscosity of the concrete produced using these fine aggregates is in the local minimum around the ideal value. It should be noted at this point that the absolute effect of matching the conductivity ratio of the fine aggregates to the viscosity of the concrete cannot (yet) be projected. However, it has been established that the viscosity has a minimum at the ideal value for the conductivity ratio.

In particular, FFi(Z1) is a conductivity ratio at which a concrete produced using fine aggregates which comprise at least 50% by mass of Z1 has a viscosity which has a local minimum, advantageously an absolute minimum, as a function of the conductivity ratio. For similar reasons to those which have already been described above, it is preferable for the ideal conductivity ratio for Z1 to be determined for a mixture of fine aggregates comprising at least 50% by mass of Z1. This can be done in a number of different ways, which will be explained below. The invention does not rule out the possibility of using mixtures containing less than 50% by mass of Z1, but the influence on the viscosity of a conductivity ratio of the fine aggregates which may deviate from the ideal value of Z1 will decrease as the level of Z1 in the fine aggregates drops. One example, for example, is to mix three sand grades, each in a proportion of around 33%, provided that the net result is a mixture with an improved conductivity ratio for each of the sand grades. According to the invention, this will have a beneficial effect on the viscosity for the mixture as a whole, and therefore for the concrete produced therewith.

It is preferable for sand grade Z1 to be selected from Table 1, column 1, and for 1/FFi (Z1) to be selected from Table 1, column 3. The ideal value FFi(Z1) has been determined for a large number of sand grades Z1 and these values have been complied in the said table. In this table, FF is actual conductivity ratio, measured on the unmodified product, and FFi is the optimum conductivity ratio according to the invention. If the viscosity has a (very) wide minimum, the ideal value for FFi or CFi is also a correspondingly wide range. After all, it is of no practical significance if the viscosity changes by just 1%, for example. Furthermore, the sand grades in the table are denoted by the standard commercial names, where in particular:

- ZZ stands for "sea sand"
- Kaliwaal 250/500 micron is fine sand from the dredging/processing vessel in the vicinity of Cuijk (Maas)
- Kaliwaal Asselt = as above, but during production (Maas) sand from Asselt is added
- Beetse is a 0/500 micron sand and a "waste product" from the Sellinger Beetse sand excavation in the north of the Netherlands
- Valewaard is a 0/500 micron sand from Valewaard (Maas)
- Plaatzand is a 0/500 micron sand and a "waste product" from a processing installation in Flushing, where sand from Great Britain is screened and washed
- Argo 0/1 is a 0/1 mm sea sand which is dredged off the cost of Flushing by the dredger ship Argo.

The inventor surmises that the following considerations may be of relevance with regard to the influence of sand grade, excavation site and the like on the (optimum) conductivity ratio, without wishing to be tied to these considerations.

The optimum CF value of the sands is determined by geological origin of the sand, the grain structure and grain shape, and the presence or absence of a chemical coating of, inter alia, oxidized or reduced iron, calcium carbonates and/or other coatings of natural origin. The following observations can made concentrated more specifically on the particular sand grade.

Meuse/Maas sands: 0/4 mm or coarser

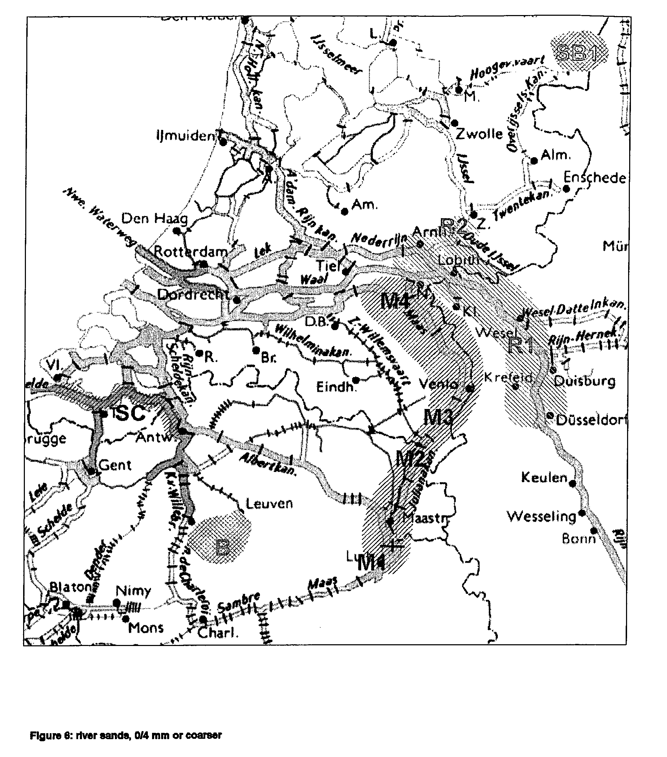

The optimum compatibility factor of the Meuse/Maas sands changes gradually from south to north, in other words downstream. The highest optimum CF value [≈ 60] is found in the alluvial plane and the terraces of the Meuse at Maaseik - Kessenich. Irrespective of this relationship, the sand from Kessenich has a second optimum CF value [6]. The optimum CF value decreases via Stevol [± 25] until Cuijk [± 15] . The actual CF values of these sands vary between 6 and 15 depending on the composition of the sand during and after excavation (extraction). The difference between these CF values and the corresponding optimum CF values decreases systematically in the downstream direction. In the region of Cuijk, the difference between the CF value of the excavated sand and the optimum CF value is virtually zero. This relationship applies to all Meuse/Maas deposits from Liege (B) to Grave (N) in the alluvial plane, low centre and high terraces of the Meuse/Maas (shading denoted by M1 to M4 in Figure 6). The sand in the alluvial plane of Liege (Amay) deviates from the above tendency since schists have been admixed with it as a result of the erosive action of the Meuse.

A type region in this context is a location where the CF value has been accurately determined and is representative of the geological deposit. The optimum CF values for locations between type regions can easily be derived by linear interpolation.

Type regions (Figure 6):

M1:   Amay
M2:   Kessenich (BM28)
M3:   Stevensweert (Stevol)
M4:   Cuijck

Meuse/Maas sands: 0/4 mm or coarser

Type regions (Figure 6):

M4:    Cuijck, listed in the tables as Kaliwaal 250/500 and Kaliwaal 250/500 Asselt
K:    Kaulille B13 20-25

Lower Rhine sands, 0/4 mm or coarser

Hitherto, the optimum compatibility factor has varied between ± 17 for the Valewaard type region in the vicinity of Doesburg aan de IJssel (northern branch of the Rhine) ± 19 for the type region Wesel am Rhein to the north of Duisburg. For the same grain structure, the CF value of the excavated sands scarcely varies between Valewaard [11] and Duisburg [9]. This relationship applies to all Lower Rhine deposits between Duisburg and he vicinity of Arnhem in the alluvial plane, low centre and high terraces of the Rhine (shading denoted by R1 and R2 in Figure 6).

Type regions (Figure 6):

R1:    Lower Rhine (Wesel)
R2:    Valewaard (Doesburg)

Lower Rhine sands, 0/4 mm or coarser

Type regions (Figure 6):

R2:    Valewaard (Doesburg), indicated in the tables as Valewaard 0/1 and VW 0-250

Other river sands, 0/2 mm or smaller

Type regions (Figure 6):

SB1:    Sellingerbeetse, referred to in the tables as Beetse
SC:    Scheldt sand, downstream of Antwerp

Sea sands, 0/4 mm or coarser

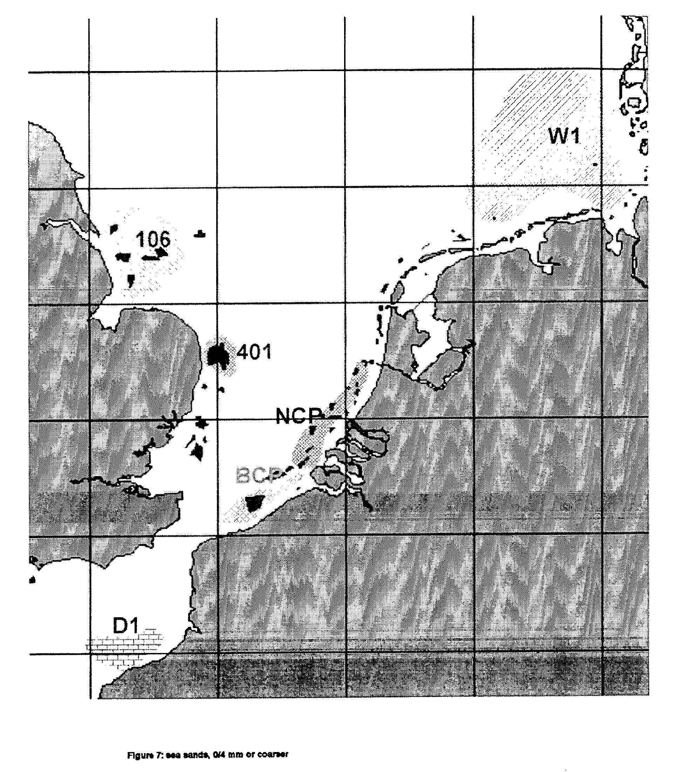

The CF value and optimum CF value of the sea sands can be determined on the sand without the salt originating from the sea water having been removed, and on the sand after desalination by washing with demineralized water. This can give rise to different combinations of sands, since the CF optimum is dependent on the salt concentrations. If the CF and CF optimum have been determined on desalinated sand, this is specifically indicated. Figure 7 indicates the geological zones for each type region:

W1:    Weißebank off the coast of Germany - Denmark
401:    Sand banks off the coast of Norwich, referred to in the tables as Plaatsand Flushing SBV and Flushing 0/2 SBV
106:    Sand banks between Skegness and Grimsby, referred to in the tables as Plaatzand and Flushing SBV and Flushing 0/2 SBV
D1:    Sand from the gravel banks off the coast of Dieppe

Sea sands 0/2 mm of fine

Type regions (Figure 7):

401: Sand banks of the coast of Norwich
106: Sand banks between Skegness and Grimsby
BCP: Belgian continental plate, referred to in the tables as Argo 0/1
NCP: Dutch continental plate, referred to in the tables as Argo 0/1 and Hook of Holland

Type region (Figure 6):

B: Sands from Brussels located in the Brussels - Waver - Leuven triangle and referred to in the tables as Chaumont Gistoux A. (These were originally fine sea deposits which can now be reclaimed on land.)

Obviously, it is also possible to determine the actual CF value as well as the usable, desired and ideal CF value(s) for other sand deposits. In this way, other type regions can also be determined.

It is advantageous for an additional fine aggregate which comprises a sand grade Z2 with a conductivity ratio Z2 to be added to Z1. A major advantage of the present invention is that the conductivity ratio of the fine aggregates, starting from sand grade Z1, can be modified by adding another sand grade Z2 with a different conductivity ratio Z2. Obviously a difference between FF(Z1) and FF(Z2) is a precondition for allowing the conductivity ratio to be changed. The advantage of the invention applies to the possibility of being able to predict the workability of concrete, or at least a change therein, on the basis of the fine aggregates used.

It is preferably the case that FF(Z2) > FF(Z1) if FFi(Z1) > FF(Z1), and FF(Z2) < FF(Z1) if FFi(Z1) < FF(Z1). If the different conductivity ratios satisfy the above conditions, the addition of the second sand grade Z2 will change the conductivity ratio of the fine aggregates as a whole towards the ideal value for sand grade Z1. In a first approximation, the inverse of the conductivity ratio of the mixture of sand grades is equal to the (mass-based) weight average of the separate inverse conductivity ratios.

However, the possibility of incorporating a second sand grade with a conductivity ratio which does not satisfy the above conditions is not ruled out. This may be the case, for example, if a higher viscosity is desired of the concrete. It would also be possible to select a sand grade with virtually the same conductivity ratio in order not to influence the viscosity.

If only the setting of the viscosity (i.e. the workability) of the concrete is important, it may be preferable to opt for a second sand grade Z2 which has a conductivity ratio which is as far as possible greater than or as far as possible lower than the conductivity ratio of Z1, if FFi(Z1) > FF(Z1) or FFi(Z1) < FF(Z1), respectively.

In still further cases, it is preferable to select a value for the conductivity ratio of sand grade Z2 which is only slightly greater or slightly lower than the conductivity ratio of Z1, in order to maximum the amount of sand grade Z2 which can be added.

The second sand grade Z2 is advantageously derived from the same excavation site as the excavation site of the first sand grade Z1, but has a different grain size distribution. It appears that the conductivity ratio of a sand grade is dependent on the grain size distribution of this sand grade. In view of this knowledge, therefore, the conductivity ratio of a sand grade Z1 can also be adjusted by adding a sand grade from the same excavation site, and therefore with the same chemical composition and the like but with a different grain size distribution. The applicant, without wishing to be tied to any particular explanation, assumes that this is to do with a different size and/or the distribution of the surface charges on the grains of the sand grade.

It is preferable for the second sand grade Z2 to comprise a sand grade obtained by comminuting grains of the first sand grade Z1. In particular by comminuting grains of the first sand grade Z1, it is possible to obtain smaller grains, in other words a different grain size distribution, which when added to the first sand grade Z1 may have a beneficial effect on the conductivity ratio thereof.

The additional fine aggregate advantageously comprises a salt. The term salt is to be understood in particular as meaning a water-soluble salt, not a super-plasticizer. A super-plasticizer is an aggregate which is added to concrete mixtures in order to lower viscosity without additional water having to be added, or alternatively to allow the quantity of water to be added to be reduced. Some examples are based on melamine formaldehyde sulphonate and certain polymers. Other examples of super-plasticizers are known to the person skilled in this field.

A major advantage of water-soluble salts is that even in small quantities they can have a considerable effect on the electrical conductivity of the mixture and therefore also on the conductivity ratio.

It is preferable for at least one salt to be at least partially removed from sand grade Z1. In most cases, this can be achieved by flushing out the salt in a solvent, in particular water. However, other methods are not ruled out, for example inactivating the salt by making it, for example, water-insoluble as a result of allowing to react with another substance.

In particular, the salt comprises an Na, K or Ca salt, preferably NaCl or KCl, or inert glauconite. In particular NaCl and KCl can advantageously be used as the salt. These salts occur naturally in sea water and therefore also in (unwashed) sea sand. It could also be added to sands obtained on land. NaCl and KCl are highly soluble and have a considerable effect on the electrical conductivity even in very small quantities.

Another salt that could be used is inert glauconite. At least two types of glauconite are known. The presence of one of these types in concrete is extremely negative, since it can cause rust etc. as a result of oxidation. For this reason, glauconite is generally removed from sand mixtures for concrete by washing or the like. However, inert glauconite, which is not oxidized in concrete, may readily be added to fine aggregates for concrete, for similar reasons to those which have been explained for NaCl and KCl.

The additional fine aggregate furthermore advantageously comprises a third sand grade Z3 with a conductivity ratio FF(Z3), in such a manner that FF(Z1) is between FF(Z3) and FFi(Z1). The addition of a third sand grade Z3 with a conductivity ratio FF(Z3) which is further away from the ideal conductivity ratio for sand grade (Z1) makes it possible to increase the proportion of sand grade Z2. Assume that the conductivity ratio of pure Z1 is already close to its ideal value, while the sand grade Z2 which it is desired to add has a conductivity ratio which is very much higher or lower than the conductivity ratio of sand grade Z1, then it is only possible for a limited quantity of sand grade Z2 to be added to sand grade Z1 while still remaining within a usable range of conductivity ratios. However, the influence of sand grade Z2 can be compensated for by adding a third sand grade Z3 with a conductivity ratio selected in such a manner that the influence of Z2 on the conductivity ratio of the fine aggregates as a whole is counteracted.

It is preferable for at least one of the sand grades to be a fine sand, with a fineness of 0/2 mm or finer. One very important advantage of the present invention is the possibility of predicting the influence of, for example, fine sand on the viscosity of concrete containing the said sand. Based on current standardization for sand for concrete, only a portion of the sand can be used for concrete. Large quantities of sand, which have hitherto in principle included any grade of fine sand, have been deemed unusable for concrete. This meant that a proportion of the sand obtained in fact represents a waste product for the concrete industry. Obviously, it will be favourable if it is possible to find a way of allowing even fine sand to be used for concrete purposes. With this in mind, extensive tests have already been undertaken, including that described in the above-referenced research report "Fine sand in concrete". According to this report, however, the viscosity will always rise, i.e. the workability will always deteriorate, when fine sand is added. This then in each case has to be compensated for by adding more water, which has an adverse effect on the strength which can be achieved, or by adding a super-plasticizer, which not only entails additional costs but also involves environmental risks, for example during the processing of concrete waste or of the super-plasticizer itself. The present invention provides a method which nevertheless allows a suitable or even better workability, i.e. a lower viscosity, to be achieved in the concrete without using these two measures. As a result, very many more grades of fine sand can be used as fine aggregate in concrete. It will be clear that this offers very considerable advantages for the efficient use of natural raw materials. This applies to a very considerable extent to use of sand obtained from sea sand. An allegation which is expressly levelled at sea sand is that the top layer, which in the North Sea frequently amounts to a layer 15 m thick, consists of sand that is unsuitable for concrete purposes. However, this sand can be made suitable for concrete purposes by simply mixing it with other sand grades.

It is advantageous for a total proportion of fine sands with a fineness of 0/2 mm in the fine aggregate to be at least 50%, preferably at least 80%. This method creates a way of composing fine aggregates which comprise a very large proportion of fine sands. In particular and advantageously, these are fine aggregates for a concrete in which a super-plasticizer is not required yet nevertheless much lower viscosity values can be obtained. These are often but not exclusively sands of different origins. It should be noted that the addition of super-plasticizers and other auxiliaries, such as retardants, is not ruled out.

In particular, a first sand grade Z1 is selected from Table 2, far left column, and a second sand grade Z2 is selected from Table 2, top row; the maximum content by mass of the second sand grade Z2 in the composed sand Z1 + Z2 is within the range of values given in the box located at the intersection of the row of Z1 and the column of Z2. It is in this way possible to form sand mixtures which give a lower viscosity of the concrete produced therewith than if the sand used were to be 100% Lower Rhine 0/5, which is known in the market as a good sand.

Even more advantageously, a first sand grade Z1 is selected from Table 3, far left column, and a second sand grade Z2 is selected from Table 3, top row, with the maximum content by mass of the second sand grade Z2 in the composed sand Z1 + Z2 being in the range of values given in the box located at the intersection of the row of Z1 and the column of Z2. It is in this way possible to form sand mixtures with which give a lower viscosity of the concrete produced therewith than if the sand used were to be 100% Cuijk 0/4, which is known in the market to be a very good sand, partly since this sand is at its own ideal value.

It should be noted that it therefore appears possible to compose sand mixtures which are better than each of the constituent sand grades, even if the latter is inherently at the ideal conductivity ratio for the corresponding sand. In general, Tables 2 and 3 indicate the possibilities of mixing two sand grades to form fine aggregates suitable for concrete. In just a single case it is not readily possible to mix two sand grades and/or to do so only has the effect of increasing viscosity. Although these combinations are not ruled out, the invention is primarily directed at combinations of sand grades which deliver a viscosity-lowering effect. Therefore Tables 2 and 3 indicate, for a number of sand grades Z1, what proportion of a sand grade Z2 is possible in order to obtain a conductivity ratio of the composed fine aggregates with which it is possible to produce concrete with a viscosity that is at most equal to concrete produced using pure Z1. In this context, the term "pure" indicates that the sand grade Z1 does not contain any additions, and also has not had one or more substances removed from it by washing or the like.

The fine aggregates advantageously comprise at least two sand grades, at least two of the sand grades, and preferably all of the sand grades, having an at least partially overlapping associated range FFb, more advantageously having an at least partially overlapping associated range FFw, and even more advantageously having an associated FFi which differs by no more than 25%, most advantageously at most 10%, from the FF of the mixture M of the fine aggregates as a whole.

If two or more sand grades have an at least partially overlapping range of conductivity ratios FFb, and preferably also an overlapping range of values for FFw, these two or more sand grades can be used, in very many different mixing ratios, to obtain a composition of fine aggregates which produce a concrete with a viscosity value which is at most equal to an otherwise identical concrete produced using fine aggregates consisting exclusively of one of the sand grades with an at least partially overlapping range of conductivity ratios.

Most advantageously, the fine aggregates comprise at least two and preferably exclusively sand grades with associated values for the ideal conductivity ratio FFi which differ by no more 10%, and obviously most preferably substantially 0%, from the fine aggregates as a whole. In this situation, the fine aggregates as a whole have a conductivity ratio which, for at least two and preferably all of the constituent sand grades, is at or at least close to the ideal value for the corresponding sand. Obviously, it is possible to select sand grades with ideal conductivity ratios which are further apart and are also further away from the conductivity ratio of the fine aggregates as a whole, but in that case the deviation of the conductivity ratio of the fine aggregates as a whole from the said ideal value or values will in relative terms have a greater effect. It should be noted there that the possibility of the fine aggregates also containing substances other than just sand grades is not ruled out.

The invention also relates to a method for composing fine aggregates for concrete, in which the fine aggregates comprise a fine sand content of at least 40%, the method comprising steps of

- selecting a first fine sand fZ1;
- adding an additional sand aZ to fZ1 in a content A, and mixing these two sands to form the fine aggregates,

in such a manner that concrete produced using cement, water, coarse aggregates and the fine aggregates in an expedient mixing ratio has a viscosity which is at most equal to the viscosity of concrete produced using cement, water, coarse aggregate and fine aggregates in substantially the same mixing ratio with the fine aggregates consisting of 100% Lower Rhine 0/4 mm sand, advantageously 100% Cuijk 0/4 mm.

Grades of concrete produced on the basis of fine aggregates consisting of Lower Rhine 0/4 mm sand are considered "well" known in the field of concrete. This means that concrete comprising this Lower Rhine sand has a more favourable workability in mixing ratios which give a defined strength. Concrete produced using Cuijk 0/4 mm sand is itself even more well known in the concrete market. The invention provides the option of composing fine aggregates on the basis of one or more sand grades, even using fine sand grades, which even without the addition of super-plasticizer have better viscosity properties than the Lower Rhine 0/4 and Cuijk 0/4 fine aggregates which are well known in the market. Although this will not be possible for every sand grade, it has been found that for various sand grades a performance of this type can indeed be obtained. This conflicts with prevailing prejudices relating to the use in particular of fine sand grades. The value of the present invention, incidentally, resides not only in the provision of formulations for usable fine aggregates for concrete, but also in the provision of the insight that it is in this way possible to use certain sand grades at all, in particular fine sand grades.

It is highly advantageously possible for all the methods according to the invention which have been described thus far to be used to compose fine aggregates which give a concrete with a viscosity that is at most equal to otherwise identical concrete based on Lower Rhine 0/4 mm or even Cuijk 0/4 mm sand. By way of example, a method of this type may comprise the measures described in claims 1 - 22, without being restricted thereto.

In addition, the invention relates to fine aggregates obtainable by a method according to the invention. In this context, consideration may be given to fine aggregates which can produce a concrete with a lower viscosity than otherwise identical concrete produced using only one of the sand grades used in the fine aggregates. These are in particular fine aggregates without super-plasticizer, although the use of a super-plasticizer is not ruled out. It should be noted at this point that it is of course also possible, for example, to add super-plasticizer to a concrete mixture rather than to the sand grades. Nevertheless, in the context of the present invention it is assumed that the concrete has been produced on the basis of fine aggregates with super-plasticizer, i.e. the instant of addition to the concrete mixture is of no relevance to the invention. The same also applies to the addition or removal of another fine aggregate to the concrete mixture. For example, it is possible first of all to mix water cement and coarse aggregates, and then to separately add two or more fine aggregates to the concrete mixture. In this case too, the fine aggregates of the concrete mixture are considered to be all such substances added to the concrete mixture, irrespective of the time at which they are added to the concrete mixture.

The fine aggregates advantageously comprise at least 40%, advantageously at least 70%, fine sand and preferably at least 40%, for preference at least 70%, fine sea sand. The fine aggregates advantageously comprise at least two sand grades, and are advantageously free of super-plasticizer, and/or a single sand grade with a modified salt or salts content, or a combination thereof. The fine aggregates in each case have a conductivity ratio which is matched to the ideal value of at least one of the constituent sand grades which forms the majority of the fine aggregates, and even more advantageously to the ideal values of two or more of the constituent sand grades.

It is highly advantageous for the fine aggregates to comprise at least two grades of fine sand from different excavation sites, forming a total proportion of the fine aggregates of at least 85%, and preferably at least 95%, which is the preferred option. The invention makes it possible to compose fine aggregates which are suitable for concrete and substantially comprise fine sand without the drawbacks which have hitherto been ascribed to the latter, namely the need to add super-plasticizer or more water.

Furthermore, the invention relates to a method for producing concrete mortar or concrete, comprising the steps of combining and mixing at least water, cement, fine aggregates and optionally coarse aggregates with a grain size coarser than 0/4 mm in an expedient mixing ratio, using fine aggregates according to the invention.

Moreover, the invention also claims exclusive rights to concrete mortar or concrete obtainable using fine aggregates according to the invention. After all, the advantage of a better workability or low viscosity manifests itself best in concrete mixtures which have actually been produced. Since there is a considerable amount of freedom in the mixing ratio of the constituent materials in the various concrete mixtures, the actual determination of the appropriate mixing ratio is left to the person skilled in the art. However, the latter will have no difficulty in determining this ratio. The following description will give a number examples. In this context too, it should be noted that fine aggregates with a suitable conductivity ratio can be composed using the method in accordance with the first aspect of the invention. The concrete produced thereby will have a viscosity which is at most equal to otherwise identical concrete produced using unmodified fine aggregates. The method can therefore be used to obtain a formulation for concrete or concrete mortar of this type. Obviously, it is in practice also possible to make mixtures with fine aggregates without looking at the conductivity ratio. The power of the present invention is of course the possibility to predict the viscosity characteristics of concrete produced using these composed fine aggregates. Since it has hitherto been assumed that mixtures of this type could only result in a deterioration in the concrete, whereas the present invention demonstrates that mixtures of this type, in particular mixtures containing fine sand grades, can in fact give concrete which is as good or even better, it should be noted at this point that exclusive rights are also claimed for concrete or concrete mortar containing fine aggregates according to the invention obtained in any other way. After all, as was made clear above, practical tests using arbitrarily selected mixtures can also give good concrete grades. However, it will be clear that in the vast majority of these cases the conductivity ratio will be more favourable than that of the constituent sand grades individually.

It is advantageous for the concrete or concrete mortar to be substantially free of super-plasticizer. At this point, it should be noted once again that a major advantage of the present invention is that the concrete can remain free of super-plasticizer, which results in environmental advantages and the like. Obviously, it remains possible to add super-plasticizer for special purposes or in order to improve the viscosity of the concrete still further. However, it is not necessary to do so.

Moreover, the invention relates to a method for measuring a conductivity ratio FF of a sand, comprising the steps of measuring a specific electrical conductivity rho-v of a substantially insulating liquid, measuring a specific electrical conductivity rho-z of a densely packed bed of the sand in the liquid, and determining FF as FF = rho-z/rho-v.

Obviously, the determination of the inverse ratio 1/FF, i.e. the ratio of the resistivities, is also covered by the method according to the invention. This value is also known as the compatibility factor (CF). This method can be used to determine the conductivity ratio of a sand, which is of importance to the present application. In this context, the term "densely packed bed" of the sand in the liquid is to be understood as meaning that the sand has been sufficiently densely packed by any desired delimiting boundary, in other words has adopted a sufficiently small overall volume in the liquid. This can be achieved, for example, by vibrating, shaking etc. It is in this way possible to obtain a relatively constant measured value for the specific electrical conductivity of this densely packed bed of the sand in the liquid. It should be noted that it is also possible to measure a bed of the sand which is not densely packed in the liquid, but it is then somewhat less easy to obtain a sufficiently constant measurement method. In practice, the degree to which the theoretically achievable density of packing is approached is an indication of the margin of error in the measurement of the specific electrical conductivity. If, for example, the packing of the sand is such that it takes up a volume which is only 1% larger than the theoretically most densely packed bed, the measured specific electrical conductivity rho-z will be a closer approximation of the theoretical values than if the packed bed has a volume which is, for example, 2% larger than the theoretically most densely packed bed.

It is advantageous for the liquid to be water, preferably demineralized water of a specific electrical conductivity of less than 0.15 milli-Siemens. In fact, there are no particular restrictions on the choice of liquid, provided only that it is substantially insulating. After all, a conductive liquid will be able to interfere with the measurement of the specific electrical conductivity of sand in the liquid. In fact, any liquid is conductive to some extent, but the specific electrical conductivity of, for example and in particular, water, advantageously demineralized water, is sufficiently low to achieve a sufficiently accurate measurement. In particular, demineralized water with a specific electrical conductivity of less than 0.15 milli-Siemens is used. Other liquids, such as various oils and the like, could also be used, however, water obviously has the advantage of being easy to use, and also has the further advantage that it is also used in the production of concrete, and consequently any side effects are acceptable.

In addition, the invention relates to a method for determining concrete viscosity as a function of a conductivity ratio FF(Z1) of a sand grade Z1, comprising the steps of:

a) measuring FF(Z1) of the sand grade;
b) producing a first concrete from cement, water, coarse aggregate and sand grade Z1, in an expedient first mixing ratio;
c) determining a viscosity of the first concrete produced;
d) changing the conductivity ratio of the sand grade by adding or removing at least one material to or from Z1;
e) measuring the conductivity ratio which has been altered in this way;
f) producing a modified concrete from cement, water, coarse aggregates and the sand grade which has been modified in this way in substantially the same mixing ratio;
g) determining the viscosity of the modified concrete in this way;
h) repeating steps d) to g) inclusive a desired number of times.

This method provides a way of determining the absolute effects on the concrete viscosity as a function of the conductivity ratio of fine aggregates, in particular of a sand grade Z1. This method as described gives a approximation of this function, the function becoming more accurate the greater the proportion of sand grade Z1 in the fine aggregates of the concrete being measured. Therefore, it is advantageous for substances which have a relatively major effect on the conductivity ratio of the fine aggregates composed in this way to be added to or removed from the sand grade Z1. It is preferable to add a salt to sand grade Z1, so that even a small added amount has a very considerable effect on the conductivity ratio. In addition, it will be possible to add a sand with a very low conductivity ratio to Z1, for example Chaumont Gistoux A, in order to enable the conductivity ratio of Z1 to be lowered as quickly as possible. In some cases, it is possible to lower the conductivity ratio by flushing one or more substances out of Z1. This will apply in particular to sea sands, from which sea salt can be washed out. Obviously, the repetition of steps d) to g) in the method is with a view to determining more than one point of the function.

Moreover, the invention relates to a method for determining a salt content in a concrete which is optimum for workability, comprising the steps of

- producing a quantity of the concrete
- determining the viscosity of the concrete as a function of the quantity of the salt added to the concrete, and
- determining the content associated with the minimum viscosity.

This method can in principle be used to determine the viscosity of the concrete as a function of the salt content in a relatively simple way. Obviously, the determination of the quantity of salt added can be linked to the determination of the conductivity ratio of the fine aggregates. It should be noted that this method applies in particular to the range of increasing conductivity ratio.

It is advantageous for salt to be substantially removed from the concrete prior to the viscosity being determined. If the concrete, and in particular any fine aggregates, for example sea sand, already contain salt, it is possible to increase the salt content range which is measured. After all, it will thus be possible to lower the basic salt content of the concrete. It will in this way also be possible to extend the range of conductivity ratios towards lower values.

In addition, the invention relates to a device for composing fine aggregates for a concrete, comprising a first feed device for supplying a first sand grade Z1 with a first conductivity ratio FF1, a second feed device for supplying a second sand grade Z2 with a second conductivity ratio FF2, a mixing device for combining Z1 and Z2 to form a sand mixture with a conductivity ratio FFm, a control means for controlling a mixing ratio Z1 and Z2 of the sand mixture, the control means comprising a measuring device which is designed to determine a conductivity ratio FFm of the sand mixture, preferably in accordance with a method according to the invention.

It is advantageous for the measuring device to comprise at least one container for holding a quantity of sand mixture that is to be measured, and a conductivity meter. A device of this type advantageously offers a possible way of making mixtures of sand grades Z1, Z2 and if appropriate also other sand grades which can provide concrete produced therewith with a desired viscosity at a desired location, but preferably at the location where the sand is excavated. Obviously, it is also possible for this device to be used at locations other than the excavation site. In this context, consideration may be given in particular to sand storage sites or also to construction sites. The details of a device according to the invention of this type can readily be realized by a person skilled in the field. By way of example, the first addition device comprises a silo or other container and, for example, a conveyer belt. There are also no particular restrictions on the mixing device. The measuring device is also not restricted to a method for measuring a conductivity ratio in accordance with the invention. Obviously, it will also be possible for the conductivity ratio to be determined elsewhere. One example of a measuring device for determining the conductivity ratio substantially need not comprise much more than a container and a conductivity or resistance meter. This is explained in more detail in the description of the examples.

It should here be noted once again that a device of this type does not have to include a measuring device, but rather a control means for controlling the mixing ratio is in principle sufficient, provided that the desired mixing ratio is known. After all, this mixing ratio does not have to have been determined using the method according to the invention. If another property, such as for example the density of the sand mixture found, is also suitable for measuring the mixing ratio which is actually achieved and comparing it with the desired mixing ratio, this can also be expedient. What is important in the invention is that mixtures of sand grades can give more suitable concrete properties than separate sand grades.

The invention will be explained in more detail below on the basis of a number of examples and the figures, in which:

Fig. 1 shows the conductivity ratio of Lower Rhine 0/4 as a function of the admixing of various grades of 0/1 mm sand;

Fig. 2 shows the influence on the workability of concrete on the basis of the mixtures shown in Fig. 1;

Fig. 3 shows the workability of concrete containing ZZ UK 0/4 mm as a function of the compatibility factor (= 1/FF);

Fig. 4 shows the workability of the concrete shown in Fig. 3 as a function of the quantity of NaCl added;

Fig. 5 diagrammatically depicts a device for composing mixtures for fine aggregates;

Fig. 6 diagrammatically depicts type regions of a number of river sands, 0/4 mm or coarser; and

Fig. 7 diagrammatically depicts type regions for a number of sea sands 0/4 mm or coarser.

First of all, some background information will be provided in particular about "fine sand". Dutch Standard NEN 5905 divides fine aggregates (for concrete) into 0/4, 0/2 and 0/1 mm sand.

The 0/4 mm sand is the main constituent of the sand if it is desired to satisfy current concrete standards. The following grain size distribution standard applies to 0/1 mm sand.

Screening residue (%) left on the screen of:

| 8 mm | 0% |
| 5. 6 mm | 0-2% |
| 4 mm | 0-15% |
| 2 mm | -- |
| 1 mm | 15-70% |
| 500 µm | -- |
| 250 µm | 80-100% |
| 125 µ | -- |

The following screening residues apply to the finer sand 0/2 and 0/1

|  | 0/1 mm sand | 0/2 mm sand |
|---|---|---|
| 8 mm | -- | -- |
| 5.6 mm | -- | -- |
| 4 mm | -- | 0% |
| 2 mm | 0% | 0-15% |
| 1 mm | 0-15% | -- |
| 500 µm | -- | 10-70% |
| 250 µm | 1-75% | -- |
| 125 µm | 60-100% | 85-100% |

In the context of the present application, 0/2 mm and finer is considered to be "fine sand". More detailed information with regard to grain size distributions for concrete sands is to be found in various handbooks, such as "Betontechnologie" [Concrete technology], ing. C. Souwerbren, 10th edition, 1998, in particular also the Dutch Concrete Technology Regulations (VTB).

The choice of sand has an influence on a number of concrete properties, such as the workability. The term workability is to be understood as representing a measure of the viscosity of concrete. There are a number of different ways of measuring this. One of these is what is known as the slump, a length by which a standardized cone of the corresponding concrete subsides under its own weight and flow properties. The higher the slump, the lower the viscosity and therefore the greater the flow properties of the concrete. Another form of measurement is a torsion measurement, which measures how much energy (for example in Ncm) is needed to start the concrete mixture moving and keep it in motion. This is fairly directly linked to the viscosity. Measurement methods of this type form part of the prior art.

The workability of concrete is an indication of, inter alia, the strength and durability which can be achieved by the concrete. In general, the higher the workability, i.e. the lower the viscosity, the better the concrete can be compacted (and therefore the higher its density), and also the lower the porosity and permeability of the set concrete. All this indicates a higher strength and a better durability. These are all relative effects.

The workability of concrete can be influenced, inter alia, by the addition of a super-plasticizer or, for example, water. However, the addition of water changes the composition of the concrete mixture and can have adverse effects on the strength and the like. Therefore, guideline values are given for the water demand of various concrete grades and for various consistency ranges or slump classes. According to these guidelines, a greater slump can only be obtained by adding a super-plasticizer but not by adding more water. Nevertheless, the importance of a good, high workability of concrete will be evident from the above.

Hitherto, it has been assumed that the addition of fine sand increased the demand for water to produce the same workability. Therefore, the addition of fine sand, even when it was permitted, was always assumed to cause a deterioration to the concrete properties. As has been described above, the present invention reveals that in many cases the opposite is the case if the conductivity ratio FF of the fine aggregates is controlled.

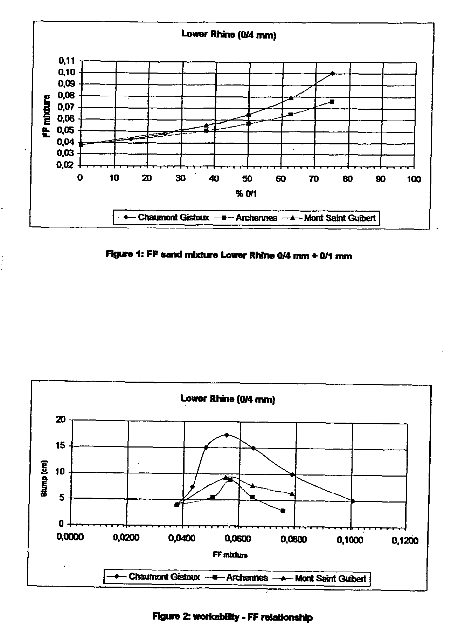

Figure 1 shows the conductivity ratio FF of Lower Rhine 0/4 sand as a function of an added quantity of fine sand 0/1 mm for three types of fine sand. It will be clearly apparent that the FF increases as the quantity of fine sand added rises, for all three of the fine sand grades. This is obviously dependent on the type of fine sand which is added.

Figure 2 plots, for at least some of the mixtures shown in Fig. 1, the workability of concrete produced using these mixtures as a function of the FF of the mixture. It can be seen from this figure that irrespective of the grade of fine sand added, the workability of concrete containing the mixture is optimum in a narrow range of FF values, with a maximum around 0.056-0.057. This lack of dependency on the cause of the change in FF was constantly observed.

Moreover, it can be seen that the absolute effect on the change of the workability does in fact differ for the fine sand grades, with a very considerable effect being achieved when Chaumont Gistoux is added. It should be noted here that it did not appear to matter which precise concrete mixture was used. Obviously, the precise, absolute influence differs, but the optimum workability was always at the same FF of the sand mixture. However, the effect is more clearly visible with less fluid concrete mixtures.

In any case, the FF value of many sand grades measured in practice varies between ± 3 and ± 100, which approximately also applies to the ideal values FFi established. In this context, it should also be noted that for some grades of sand there is more than one value for FF which has a local maximum in the workability. This is then indicated in the table as a collection of values. It is as yet unclear whether this is caused by the fact that the sand is a mixture of a plurality of geological deposits or by other factors.

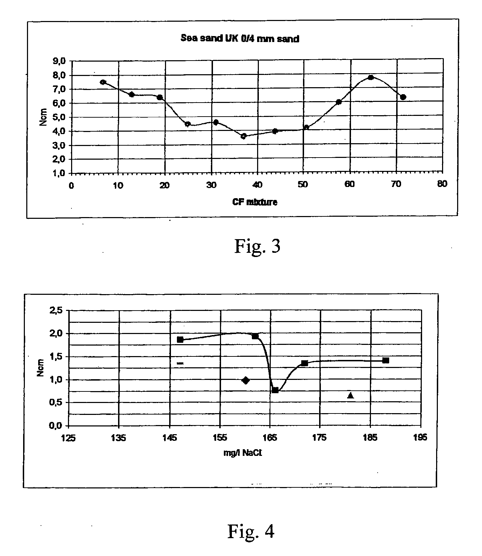

Fig. 3 and 4 give results for a different way of adapting the FF to the ideal value for the particular sand grade. This method makes use of the removal or addition of salts, in particular NaCl.

Fig. 3 plots the workability of a concrete obtained using sea sand UK 0/4 mm against the compatibility factor CF (= 1/FF) of the mixture, in this case of the sand itself with added salt. In this case, the sand may have been desalinated in advance in order to obtain a wider measurement range. It can be seen from the graph that there is a minimum around a CF of ± 40, i.e. FF of ± 0.025.

Fig. 4 once again plots the workability of a concrete obtained using the said sand, in this case as a function of the quantity of NaCl added. It can be seen that the viscosity has a clear minimum at a value of ± 165 mg/l of NaCl. If, therefore, this quantity of NaCl is added to these fine aggregates, a viscosity which is only approximately one half to one third of the viscosity of mixtures containing a NaCl contamination which differs by just 5% is achieved. This sensitivity to the salt content corresponds to practical experience where, for example, certain grades of sea sand which were inherently suitable for concrete were subsequently washed. The workability was found to deteriorate dramatically and suddenly. Given knowledge of the present invention, this can be attributed to a drastic change in the FF.

It should also be noted there that the moment at which NaCl is added is of no importance. By way of example, it is possible for it to be added to the sand itself or to the concrete mixture or to the water, etc. What is important is that the salt content of the concrete mixture, based on the fine aggregates, has a defined value.

Another example of matching the conductivity ratio of fine aggregates relates to optimizing the grain size distribution. As has been described, each grain size (distribution) in principle has its own conductivity ratio. In some cases, therefore, it is possible to combine different fractions in an expedient distribution, in such a manner that the mixture as far as possible approaches the ideal conductivity ratio. An example which has been implemented in practice is the case of Valewaard 0/4, to which the following applies:

$$(25.20 \cdot f_1) + (12.27 \cdot f_2) + (7.45 \cdot f_3) + (9.4 \cdot f_4) + (9.35 \cdot f_5) = 10.85$$

in which

$f_1$ = 0-250 micron content,
$f_2$ = 250-500 micron content
$f_3$ = 500-1000 micron content
$f_4$ = 1.0-2.0 mm content
$f_5$ = 2.0-5.0 mm content,

and the number before the contents in each case relates to the compatibility factor (= 1/FF) of this fraction.

As soon as this is complied with, the workability is found to be (approximately) the same, with the compressive strength retained. By way of example, $f_1$-$f_5$ contents of (approximately) 6.7%, 35%, 31.3%, 17% and 10% are suitable. Obviously, these values will have to be adapted for other fraction boundaries. In addition, it is possible to add other sand grades.

A measuring appliance for measuring the conductivity ratio may simply comprise a measurement cell, such as an open tray with, for example, two plate electrodes at in principle any desired but fixed distance, between which the sand/water mixture can be placed. The conductivity ratio can then be determined with the aid of a resistance or conductivity meter by measuring the electrical conductivity of the sand/water mixture and measuring the electrical conductivity of the water, and dividing the values by one another. In doing so, it is possible, for example, to follow standard BS 1377 Part 3: "Measurement of Resistivity - Open Container Method".

The sand/water mixture should preferably be vibrated to its greatest density in order to obtain reproducible measurements. Furthermore, the measurements should preferably take place at 20°C or should at least be calculated back to this temperature.

The distilled water has a resistivity from approximately 7000 Ωm to more than 20 000 Ωm. The effect of this is eliminated by the division used to arrive at the conductivity ratio, which is a dimensionless number that is independent of the resistivity of the water or the liquid in general.

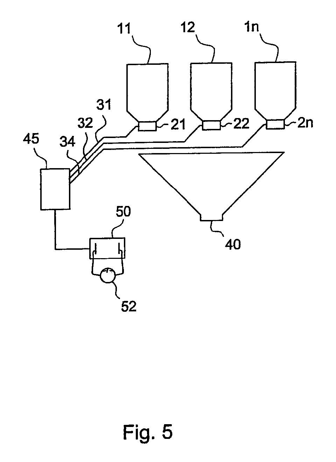

Fig. 5 diagrammatically depicts a device for composing mixtures for fine aggregates. In this figure, 11, 12, ..., In are containers of various sand grades and/or other fine aggregates (for example salts). 21, 22, ..., 2n are shut-off valves which are designed to dispense a defined quantity of fine aggregate from the associated vessel at a dispensing opening 40. For this purpose, the shut-off valves are connected to a central processing unit 45 by means of communication lines (31, 32, ..., 3n.

A measurement cell 50 with a resistance or conductivity meter 52 is also connected to the central processing unit 45. However, this is optional.

The number of vessels 11, ..., In is in principle at least 2, but there is no maximum. A practical number will be between 4 and 20, for example, but hard limits cannot and need not be set. The vessels 11, ... , 1n may be silos, ships' holds or the like, but may also simply be piles of sand. It is also possible for sand grades to be taken directly from the excavation device, for example from cyclones.

The central processing unit 45 may, for example, comprise a computer with a memory. The computer memory can be used, for example, to store data relating to the various aggregates, such as actual conductivity ratio, grain size distribution and density. The unit 45 can then operate the shut-off valves 21, ..., 2n on the basis of this data in order to compose the desired mixture of fine aggregates, which can be dispensed via dispensing opening 40 to, for example, a lorry or cargo ship.

The optional measurement cell 50 with measurement device 52 can be provided, for example, if an actual conductivity ratio of a sand grade still needs to be determined, for example when using new sand grades. If, incidentally, the data of a sand grade are already known, it is also possible to make do, for example, with a density measurement or a simple "formulation" to compose a mixture of fine aggregates with sufficient accuracy.

| 0/4 mm sands or coarser | 1/FFz1 CFz1 | 1/FFb CFb | 1/FFi CFi | 1/FFw CFw |
|---|---|---|---|---|
| | | | | (w = ± 10%) |
| Lower Rhine 0/5 | 8.68 | 8.11--28.0 | 8.20 & 15.0 | 8.12-25 |
| 0/4 Valewaard | 10.9 | 11 - 23 | 16.9 | 13 - 21 |
| Amay 0/4 July 2003 | 12.1 | 10 - 100 | 6 & 60 | 10 - 100 |
| Meuse Kessenich BM28 | 13.8 | 10 - 40 | 14 | 10 - 40 |
| Maas sand 0/4 mm Cuijk | 15.0 | 5 - 15 | 15.0 | 7 - 13 |
| Sea sand 0/4 mm UK 106-401, desalinated | 27 - 37 | 15 - 55 | 35 - 53 | 20 - 50 |
| Sea sand 0/5 mm Weissebank | 50 | 12 - 68 | 25 - 65 | 14 - 65 |
| Sea sand 0/4 mm UK 106-401 | 70 | 50 - 71 | 70 | 55 - 70 |
| Sea sand 0/5 Dieppe | 75 | 10 - 75 | 34 | 10 - 70 |

| 0/2 mm sand or finer | | | | |
|---|---|---|---|---|
| Chaumont Gistoux A | 5 | | | |
| Beeste, washed in laboratory | 6 | | | |
| Beetse 0/500 mu 2003 | 6 | | | |
| Beetse, washed in quarry | 7 | | | |
| Kaliwaal 250/500 | 7 | | | |
| Kaliwaal 250/500 Asselt | 8 | | | |
| Hook of Holland | 12 | | | |
| Valewaard 0/1 | 12 | | | |
| Beetse, unwashed | 17 | | | |
| VW 0-250 | 23 | | | |
| Scheldt sand | 33 | | | |
| Kaulille B13 20-25 | 45 | | | |
| Flushing 0/2 SBV | 57 | | | |
| Plaatzand Flushing SBV | 58 | | | |
| Wezendonk | 59 | | | |
| Argo 0/1 | 71 | | | |

Table 1 conductivity ratios for a number of sand grades

| Admixture [%] | Kaliwaal 250/500 | Chaumont Gistoux A | Chaumont Gistoux C | Beetse 0/500 mu | Kaliwaal 250/500 Asselt | Valewaard 0/500 | Plaatzand | Wezendonk | Scheldt sand | Argo 0/1 |
|---|---|---|---|---|---|---|---|---|---|---|
| Lower Rhine 0/5 | 55 | | | | 55 | 45 | | | | 35 |
| Meuse Kessenich (BM28) | | 100 | | | 80 | 35 | 65 | | | 85 |
| Cuijk | | 100 | | | | | | | 55 | 80 |
| 0/4 Valewaard | | | | | | | | | | 10 |
| 0/4 mm Amay (2003) | | 60 | | | 100 | | | | | 60 |
| Stevol | 50 | | | | 50 | | | | | |
| ZZ 0/5 UK 409 (Hanson, SBV desalinated) | | | | | | | | | | |
| ZZ 0/5 UK 106 (Hanson EdP) | | | | | | | | | | |
| ZZ 0/5 UK 106 (Hanson EdP) desalinated | | | | | | | | | | |
| ZZ 0/5 Weiße Bank DEME | | | | | | | | | | |

Table 2, maximum admixture of sands with respect to Lower Rhine 0/5

| Admixture [%] | Kaliwaal 250/500 | Chaumont Gistoux A | Chaumont Gistoux C | Beetse 0/500 mu | Kaliwaal 250/500 Asselt | Valewaard 0/500 | Plaatzand | Wezendonk | Scheldt sand | Argo 0/1 |
|---|---|---|---|---|---|---|---|---|---|---|
| Lower Rhine 0/5 | | 25 | 25 | 25 | | | | 20 | 42 | |
| Meuse Kessenich (BM28) | | | | | | | | | | |
| Cuijk | | | | | | | | | | |
| 0/4 Valewaard | | | | | | | | | | |
| 0/4 mm Amay (2003) | | | | | | | | | | |
| Stevol | 35. | | | | 35 | | | 37 | | |
| ZZ 0/5 UK 409 (Hanson, SBV desalinated) | 30 | 55 | | | 30 | | | | 25 | 40 |
| ZZ 0/5 UK 106 (Hanson EdP) | | | | | | | | | 5 | 0 |
| ZZ 0/5 UK 106 (Hanson EdP) desalinated | | | | | | | | | 35 | 65 |
| ZZ 0/5 Weiße Bank DEME | 70 | 75 | | | 70 | | | | | 80 |

Table 3 maximum admixture of sands with respect to Cuijk 0/4

**Claims**

1. Method for composing a mixture M of fine aggregates for concrete,
in which the fine aggregates comprise one or more sand grades with a grain size of substantially 0/4 mm or below and zero or more optionally dissolved salts, excluding cement,
the method comprising the steps of

   - providing a first sand grade Z1,
   - determining a conductivity ratio FF(Z1) of Z1, the conductivity ratio being the mathematical ratio between a specific electrical conductivity of a substantially insulating liquid and a specific electrical conductivity of the sand grade in this liquid,
   - selecting a range of values for usable conductivity ratios FFb(Z1) associated with the sand grade Z1,
   - matching a composition of the fine aggregates to a mixture M with a conductivity ratio FF(M), by adding or removing at least one additional fine aggregate to or from the sand grade Z1,

   in a ratio which is such that the FF(M) of the mixture M is within the selected range of values of usable conductivity ratios FFb(Z1).

2. Method according to claim 1, in which FFb(Z1) is determined as the range of values of usable conductivity ratios in which the viscosity of concrete produced using fine aggregates which comprise at least 50% by mass of Z1 is at most equal to the viscosity of an otherwise identical concrete produced using fine aggregates which consist exclusively of Z1.

3. Method according to one of the preceding claims, in which the sand grade Z1 is selected from Table 1, column 1, and FFb(Z1) is selected from Table 1, column 3.

4. Method according to claim 1, in which the difference between FF(M) of the mixture and a predetermined range of values of desired conductivity ratios FFw(Z1) associated with sand grade Z1 is at most equal to and is advantageously lower than the difference between FF(Z1) and FFw(Z1), and is even more advantageously virtually 0.

5. Method according to claim 1, in which FFw(Z1) is the range of values of desirable conductivity ratios in which the viscosity of a concrete produced using fine aggregates which comprise at least 50% by mass of Z1 is at least 10%, and preferably at least 25%, lower than the viscosity of an otherwise identical concrete produced using fine aggregates which consist exclusively of Z1.

6. Method according to one of claims 4-5, in which sand grade Z1 is selected from Table 1, column 1, and 1/FFw(Z1) is selected from Table 1, column 4.

7. Method according to one of the preceding claims, in which the difference between FF(M) of the mixture and a predetermined ideal value of the conductivity ratio FFi(Z1) associated with sand grade Z1 is at most equal to, and advantageously lower than, the difference between FF(Z1) and FFi(Z1).

8. Method according to claim 7, in which the difference between FF(M) and FFi(Z1) is less than 5% of FFi(Z1).

9. Method according to claim 7 or 8, in which FFi (Z1) is a conductivity ratio at which a concrete produced using fine aggregates which comprise at least 50% by mass of Z1 has a viscosity which has a local minimum as a function of the conductivity ratio.

10. Method according to one of claims 7-9, in which sand grade Z1 is selected from Table 1, column 1, and 1/FFi(Z1) is selected from Table 1, column 3.

11. Method according to one of the preceding claims, in which an additional fine aggregate which comprises a sand grade Z2 with a conductivity ratio Z2 is added to Z1.

12. Method according to claim 11, in which FF(Z2) > FF(Z1) if FFi(Z1) > FF(Z1), and FF(Z2) < FF(Z1) if FFi(Z1) < FF(Z1).

13. Method according to one of claims 11-12, in which the second sand grade Z2 originates from the same excavation site as the excavation site of the first sand grade Z1, but has a different grain size distribution.

14. Method according to claim 13, in which the second sand grade Z2 comprises a sand grade obtained by comminuting grains of the first sand grade Z1.

15. Method according to one of the preceding claims, in which the additional fine aggregate comprises a salt.

16. Method according to one of the preceding claims, in which at least one salt is at least partially removed from sand grade Z1.

17. Method according to claim 15 or 16, in which the salt comprises an Na, K or Ca salt, preferably NaCl or KCl, or inert glauconite.

18. Method according to one of claims 11-17, in which the additional fine aggregate also comprises a third sand grade Z3 with a conductivity ratio FF(Z3), such that FF(Z1) is between FF(Z3) and FFi (Z1).

19. Method according to one of the preceding claims, in which at least one of the sand grades is a fine sand, with a fineness of 0/2 mm or finer.

20. Method according to one of the preceding claims, in which a total proportion of fine sands with a fineness of 0/2 mm in the fine aggregates amounts to at least 50%, preferably at least 80%.

21. Method according to one of claims 11-20, in which a first sand grade Z1 is selected from Table 2, far left column, and a second sand grade Z2 is selected from Table 2, top row, the content by mass of the second sand grade Z2 in the composed sand Z1 + Z2 amounting to at most the value in the box located at the intersection of the row of Z1 and the column of Z2, and advantageously a first sand grade Z1 is selected from Table 3, far left column, and a second sand grade Z2 is selected from Table 3, top row, the content by mass of the second sand grade Z2 in the composed sand Z1 + Z2 amounting to at most the value in the box located at the intersection of the row of Z1 and the column of Z2.

22. Method according to one of the preceding claims, in which the fine aggregates comprise at least two sand grades, at least two of the sand grades, and preferably all of the sand grades, having an at least partially overlapping associated range FFb, advantageously having an at least partially overlapping associated range FFw, and more advantageously having an associated FFi which differs by no more than 10% from the FF of the mixture M of the fine aggregates as a whole.

23. Method for composing fine aggregates for concrete, in which the fine aggregates comprise a fine sand content of at least 40%, the method comprising steps of

   - selecting a first fine sand fZ1;
   - adding an additional sand aZ to fZ1 in a content A, and mixing these two sands to form the fine aggregates,

   in such a manner that concrete produced using cement, water, coarse aggregates and the fine aggregates in an expedient mixing ratio has a viscosity which is at most equal to the viscosity of concrete produced using cement, water, coarse aggregate and fine aggregates in substantially the same mixing ratio with the fine aggregates consisting of 100% Lower Rhine 0/4 mm sand, advantageously 100% Cuijk 0/4 mm.

24. Fine aggregates obtainable by a method according to one of the preceding claims.

25. Fine aggregates according to claim 24, comprising at least 40%, advantageously at least 70%, fine sand.

26. Fine aggregates according to claim 24 or 25, comprising at least 40%, advantageously at least 70%, fine sea sand.

27. Method for producing concrete mortar or concrete, comprising the steps of combining and mixing at least water, cement, fine aggregates and optionally coarse aggregates with a grain size coarser than 0/4 mm in an expedient mixing ratio, using fine aggregates according to one of claims 24-26.

28. Concrete or concrete mortar obtainable by a method according to claim 27.

29. Concrete or concrete mortar according to claim 28, which is substantially free of super-plasticizer.

**30.** Method for measuring a conductivity ratio FF of a sand, comprising the steps of measuring a specific electrical conductivity rho-v of a substantially insulating liquid, measuring a specific electrical conductivity rho-z of a densely packed bed of the sand in the liquid, and determining FF as FF = rho-z/rho-v.

**31.** Method according to claim 30, in which the liquid is water, preferably demineralized water with a specific electrical conductivity of less than 0.15 milli-Siemens.

**32.** Method for determining concrete viscosity as a function of a conductivity ratio FF(Z1) of a sand grade Z1, comprising the steps of:

a) measuring FF(Z1) of the sand grade;
b) producing a first concrete from cement, water, coarse aggregate and sand grade Z1, in an expedient first mixing ratio;
c) determining a viscosity of the first concrete produced;
d) changing the conductivity ratio of the sand grade by adding or removing at least one material to or from Z1;
e) measuring the conductivity ratio which has been altered in this way;
f) producing a modified concrete from cement, water, coarse aggregates and the sand grade which has been modified in this way in substantially the same mixing ratio;
g) determining the viscosity of the modified concrete in this way;
h) repeating steps d) to g) inclusive a desired number of times.

**33.** Device for composing fine aggregates for a concrete, comprising a first feed device for supplying a first sand grade Z1 with a first conductivity ratio FF1, a second feed device for supplying a second sand grade Z2 with a second conductivity ratio FF2, a mixing device for combining Z1 and Z2 to form a sand mixture with a conductivity ratio FFm, a control means for controlling a mixing ratio Z1 and Z2 of the sand mixture, the control means comprising a measuring device which is designed to determine a conductivity ratio FFm of the sand mixture, preferably in accordance with claim 27 or 28.

**34.** Device according to claim 33, in which the measuring device comprises at least one container for holding a quantity of sand mixture that is to be measured, and a conductivity meter.

Figure 1: FF sand mixture Lower Rhine 0/4 mm + 0/1 mm

Figure 2: workability - FF relationship

Fig. 3

Fig. 4

Fig. 5

Figure 6: river sands, 0/4 mm or coarser

Figure 7: sea sands, 0/4 mm or coarser